# EUROPEAN PATENT APPLICATION

(11) **EP 4 624 935 A1**
(43) Date of publication of application: **01.10.2025**
(21) Application number: 24223606.5
(22) Date of filing: 30.12.2024
(51) Int. Cl.: G01N 35/04

(54) **SAMPLE ANALYSIS SYSTEM**

(30) Priority: 31.03.2024 CN 202410394332
(71) Applicant: Shenzhen Mindray Bio-Medical Electronics Co., Ltd., Shenzhen, Guangdong 518057 (CN)
(72) Inventor: ZHANG, Kun, Shenzhen, 518057 (CN); GAO, Feng, Shenzhen, 518057 (CN)
(74) Representative: KIPA AB

(57) **Abstract**

Provided is a sample analysis system. The sample analysis system includes a first analysis module, a second analysis module and a transit mechanism. The first analysis module includes a first dispatching component and a first test component. The second analysis module includes a second dispatching component and a second test component. The transit mechanism is formed with a transit area, wherein the second dispatching mechanism is further configured to dispatch first and/or second sample vessels between the transit area and the second analysis module, and the first dispatching mechanism is further configured to dispatch first and/or second sample vessels between the first analysis module and the transit area.

## Description

### TECHNICAL FIELD

The present application relates to the technical field of medical devices, and in particular to a sample analysis system.

### BACKGROUND

In order to improve a sample test throughput, multiple sets of sample feeding mechanisms and multiple sets of test mechanisms are usually provided in a sample analysis system, and test is performed by using multiple sets of test mechanisms, which may effectively improve a sample test efficiency.

Test items based on different samples may be different. For example, some of the samples may need to be tested in one of the test mechanisms, some of the samples may need to be tested in the other one of the test mechanisms, and some of the samples may need to be tested in both the test mechanisms. Therefore, in order to improve test efficiency, it usually needs to load a corresponding sample in a specified channel.

However, the sample analysis system is provided with multiple sample feeding mechanisms, and therefore a situation where the corresponding sample is not loaded in the specified channel may occur. In the related art, if a sample is loaded in a wrong sample feeding mechanism, manual-assisted dispatching is needed. Therefore, the sample analysis system still needs to be optimized to further improve the sample test efficiency.

### SUMMARY

A main object of embodiments of the present application is to provide a sample analysis module and a sample analysis system.

According to a first aspect, an embodiment of the present application provides a sample analysis system, the sample analysis system includes a first analysis module, a second analysis module and a transit mechanism.

The first analysis module includes a first dispatching component and a first test component, wherein the first dispatching component comprises a first sample feeding mechanism and a first dispatching mechanism, the first sample feeding mechanism is configured for a user to place one or more first sample vessels that each first sample vessel contains a first sample, the first test component is configured to test at least a portion of the first sample in each of at least a portion of the first sample vessels provided by the first sample feeding mechanism, so as to obtain a first test result, and the first dispatching mechanism is configured to dispatch, in the first analysis module, at least a portion of the first sample vessels provided by the first sample feeding mechanism, so as for the first test component to test at least a portion of the first sample in each of at least a portion of the first sample vessels provided by the first sample feeding mechanism, to obtain the first test result.

The second analysis module includes a second dispatching component and a second test component, wherein the second dispatching component comprises a second sample feeding mechanism and a second dispatching mechanism, the second sample feeding mechanism is configured for the user to place one or more second sample vessels that each second sample vessel contains a second sample, the second test component is configured to test at least a portion of the second sample in each of at least a portion of the second sample vessels provided by the second sample feeding mechanism, so as to obtain a second test result, and the second dispatching mechanism is configured to dispatch, in the second analysis module, at least a portion of the second sample vessels provided by the second sample feeding mechanism, so as for the second test component to test at least a portion of the second sample in each of at least a portion of the second sample vessels provided by the second sample feeding mechanism, to obtain the second test result, the first dispatching component and the second dispatching component dispatch respective sample vessels independently of each other.

The transit mechanism is formed with a transit area, wherein the second dispatching mechanism is further configured to dispatch first and/or second sample vessels between the transit area and the second analysis module, and the first dispatching mechanism is further configured to dispatch first and/or second sample vessels between the first analysis module and the transit area.

As may be seen from the above technical solution provided in the application, the sample analysis system provided in the application is provided with at least two sample feeding mechanisms, at least two dispatching mechanisms and at least two test mechanisms. Each of the sample feeding mechanisms, each of the dispatching mechanisms and each of the test mechanisms may cooperate with each other to form a set of analysis module which may analyze samples independently, and two sets of analysis modules may form two analysis modules respectively, thereby forming the sample analysis system. Furthermore, the sample analysis system is provided with a transit mechanism, sample vessels are dispatched between the transit area and the second dispatching component by using the second dispatching mechanism, and the sample vessels are dispatched between the first dispatching component and the transit area by using the first dispatching mechanism, such that a sample loaded from the first analysis module may enter the second analysis module for test, or the sample loaded from the second analysis module may also enter the first analysis module for test, such that in case that dispatching of sample vessels between the first analysis module and the second analysis module is need to test a sample in a corresponding target analysis module, manual dispatching is not needed, and accurate and efficient dispatching of the sample during test is further achieved, which further improves test efficiency of the sample and makes a product use experience better.

It should be understood that the above general descriptions and the following detailed descriptions are only exemplary and explanatory, and cannot limit disclosures of the embodiments of the present application.

### BRIEF DESCRIPTION OF THE DRAWINGS

In order to explain technical solutions of the embodiments of the present application more clearly, drawings required to be used in descriptions of the embodiments will be briefly introduced below. It is apparent that the drawings in the following descriptions are some embodiments of the present application, and other drawings may also be obtained by those of ordinary skill in the art based on these drawings without paying any creative work.
FIG. 1 is a schematic diagram of a block structure of a sample analysis system provided in some implementations.
FIG. 2 is a schematic structural diagram of dispatching a sample between two different analysis modules by a sample analysis system provided in some implementations through a cascade dispatching mechanism.
FIG. 3 and FIG. 4 are schematic structural diagrams of dispatching a sample vessel between two different analysis modules by a cascade dispatching mechanism of a sample analysis system provided in some implementations using a buffer area.
FIG. 5 to FIG. 8 are schematic structural diagrams of dispatching a sample vessel between two different analysis modules by a cascade dispatching mechanism of a sample analysis system provided in some implementations using a transit area.
FIG. 9 is a schematic structural diagram of dispatching a sample vessel between sample feeding channels of two different analysis modules by two dispatching mechanisms of a sample analysis system provided in some implementations.
FIG. 10 is a schematic structural diagram of transferring a sample vessel between two analysis modules of a sample analysis system provided in some implementations through respective dispatching mechanisms, to dispatch the sample vessel.
FIG. 11 to FIG. 13 are schematic structural diagrams of dispatching a sample vessel by two analysis modules of a sample analysis system provided in some implementations through independent transit mechanisms.
FIG. 14 and FIG. 15 are schematic diagrams of frame structures of a sample analysis module provided in some implementations.

### DETAILED DESCRIPTION

Hereinafter, technical solutions in implementations of the present application will be clearly and completely described with reference to drawings in the implementations of the present application. It is apparent that the described embodiments are part of the embodiments of the present application, rather than all of the embodiments. Based on the embodiments in the present application, all other embodiments obtained by those of ordinary skill in the art without paying any creative work shall fall within the scope of protection of the present application.

In descriptions of the present application, unless otherwise specified and defined explicitly, terms "install", "connected to" and "connect" should be understood in a broad sense, for example, they may be fixed connection, detachable connection, or integral connection; they may be mechanical connection or electrical connection; they may be direct connection, or indirect connection through an intermediate medium, or may be communication between interiors of two elements. Specific meanings of the above terms in the present application may be understood by those of ordinary skill in the art according to a specific situation.

Flowcharts shown in the drawings are only exemplary explanations, and do not necessarily include all contents and operations/steps, nor are they necessarily performed in described orders. For example, some operations/steps may also be decomposed, combined or partially merged, therefore orders performed actually may be changed according to an actual situation.

Hereinafter, some implementations of the present application will be described in detail with reference to the drawings, and features in the following embodiments and implementations may be combined with each other without conflict.

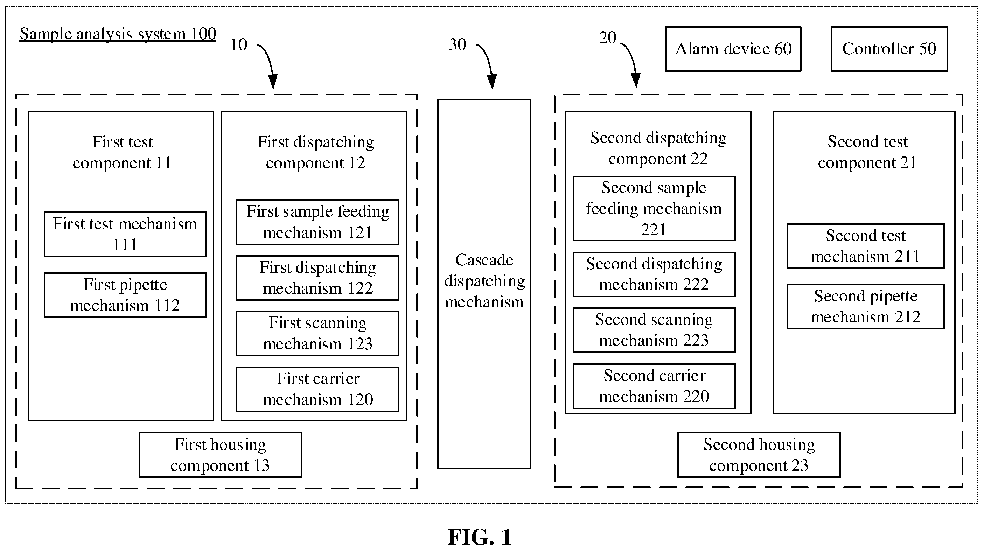

With reference to FIG. 1, FIG. 1 is a schematic diagram of a frame structure of a sample analysis system provided in an embodiment of the present application.

As shown in FIG. 1, the sample analysis system 100 includes a first analysis module 10 and a second analysis module 20. Both the first analysis module 10 and the second analysis module 20 are configured to analyze a sample under test, to obtain a corresponding test result.

In some implementations, the first analysis module 10 includes, but is not limited to at least one of a biochemical analyzer, an immunoanalyzer, a coagulation analyzer, and a urine analyzer; the second analysis module 20 includes, but is not limited to at least one of a biochemical analyzer, an immunoanalyzer, a coagulation analyzer, and a urine analyzer.

It may be understood that the first analysis module 10 and the second analysis module 20 may be the same sample analysis module, or may be different sample analysis modules, which is not limited here.

For example, the first analysis module 10 is a biochemical analyzer configured to perform biochemical item test on the sample under test, and the second analysis module 20 is an immunoanalyzer configured to perform immunization item test on the sample under test. For another example, both the first analysis module 10 and the second analysis module 20 are biochemical analysis modules configured to perform biochemical item test on the sample respectively, or both the first analysis module 10 and the second analysis module 20 are immunoanalyzers configured to perform immunization item test on the sample respectively.

As shown in FIG. 1, in some implementations, the sample analysis system 100 further includes a cascade dispatching mechanism 30 configured to dispatch sample vessels between the first analysis module 10 and the second analysis module 20.

Specifically, the first analysis module 10 includes a first dispatching component 12 and a first test component 11. The first dispatching component 12 is configured to dispatch at least a portion of sample vessels loaded from the first analysis module 10 to the first test component 11, so as for the first test component 11 to test at least a portion of the sample in each of at least a portion of the sample vessels provided by the second sample feeding mechanism to obtain a first test result. The second analysis module 20 includes a second dispatching component 22 and a second test component 21. The second dispatching component 12 is configured to dispatch at least a portion of sample vessels loaded from the second analysis module 20 to the second test component 21, so as for the second test component 21 to perform test to obtain a second test result of the sample.

The first dispatching component 12 includes a first sample feeding mechanism 121, a first dispatching mechanism 122 and a first carrier mechanism 120, the first sample feeding mechanism 121 is configured for a user to place one or more sample vessels that each sample vessel contains a sample, the first test component 11 is configured to test at least a portion of the sample in each of at least a portion of the sample vessels provided by the first sample feeding mechanism 121 to obtain a first test result, and the first dispatching mechanism 122 is configured to dispatch, in the first analysis module 10, at least a portion of sample vessels provided by the first sample feeding mechanism 121, and dispatch the sample vessels to the first carrier mechanism 120, so as for the first test component 11 to test at least a portion of the sample in each of at least a portion of the sample vessels provided by the first sample feeding mechanism 121 to obtain the first test result.

The second dispatching component 22 includes a second sample feeding mechanism 221, a second dispatching mechanism 222 and a second carrier mechanism 220, the second sample feeding mechanism 221 is configured for the user to place one or more sample vessels that each sample vessel contains a sample, the second test component 21 is configured to test at least a portion of the sample in each of at least a portion of the sample vessels provided by the second sample feeding mechanism 221 to obtain a second test result, and the second dispatching mechanism 222 is configured to dispatch, in the second analysis module 20, at least a portion of sample vessels provided by the second sample feeding mechanism 221, and dispatch the sample vessels to the second carrier mechanism 220, so as for the second test component 21 to test at least a portion of the sample in each of at least a portion of the sample vessels provided by the second sample feeding mechanism 221 to obtain the second test result.

Furthermore, the first dispatching component and the second dispatching component may dispatch the sample vessels independently of each other. The cascade dispatching mechanism 30 is configured to dispatch at least a portion of the sample vessels provided by the first sample feeding mechanism 121 to the second analysis module 20, so as for the second test component 21 to test at least a portion of the sample in each of the sample vessels dispatched by the cascade dispatching mechanism 30, to obtain the second test result. And/or, the cascade dispatching mechanism 30 is configured to dispatch at least a portion of the sample vessels provided by the second sample feeding mechanism 221 to the first analysis module 10, so as for the first test component 11 to test at least a portion of the sample in each of the sample vessels dispatched by the cascade dispatching mechanism 30, to obtain the second test result.

Optionally, the first dispatching component 12 further includes a first scanning mechanism 123 configured to scan the sample vessels provided by the first sample feeding mechanism 121, to acquire sample information corresponding to the sample carried by each of the sample vessels, and the sample information includes at least a test item of the sample.

Optionally, the second dispatching component 22 further includes a second scanning mechanism 223 configured to scan the sample vessels provided by the second sample feeding mechanism 221, to acquire sample information corresponding to the sample carried by each of the sample vessels, and the sample information includes at least a test item of the sample.

In some implementations, the cascade dispatching mechanism 30 may be a third dispatching mechanism independent of the first dispatching mechanism 112 and the second dispatching mechanism 222, that is, the cascade dispatching mechanism 30, the second dispatching mechanism 222 and the first dispatching mechanism 112 may dispatch the sample vessels independently of one another.

Optionally, the cascade dispatching mechanism 30 may be arranged in the first analysis module 10, or may be arranged in the second analysis module 20, or may be partially arranged in the first analysis module 10 and partially arranged in the second analysis module 20.

Optionally, the cascade dispatching mechanism 30 is arranged independently of the first analysis module 10 and the second analysis module 20, and it is only required that the sample vessels may be dispatched between the first analysis module 10 and the second analysis module 20.

Optionally, the first dispatching mechanism 122 includes, but is not limited to at least one of a dispatching trolley, a mechanical gripper, and a conveyer. Optionally, the second dispatching mechanism 222 includes, but is not limited to at least one of a dispatching trolley, a mechanical gripper, and a conveyer. Optionally, the cascade dispatching mechanism 30 includes, but is not limited to at least one of a dispatching trolley, a mechanical gripper, and a conveyer.

In some implementations, at least one of the first dispatching mechanism 112 and the second dispatching mechanism 222 may also be also used as the cascade dispatching mechanism 30. That is, at least one of the first dispatching mechanism 112 and the second dispatching mechanism 222 may also be used as the cascade dispatching mechanism.

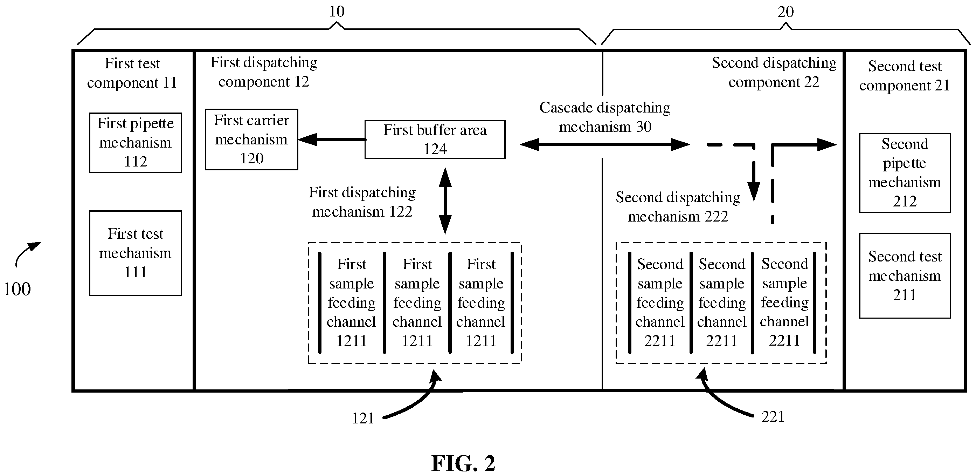

With reference to FIG. 2, exemplarily, the sample under test is placed in the sample vessel to be transported or stored through the sample vessel, and when the user needs to analyze the sample by the sample analysis system 100, the user only needs to place the sample vessel carrying the sample under test in the first sample feeding mechanism 121 corresponding to the first analysis module 10 or the second sample feeding mechanism 221 corresponding to the second analysis module 20. For example, one or more sample vessels may be placed in a sample holder, and the sample vessels are placed in the sample feeding mechanism of the analysis module through the sample holder.

After the sample vessel is loaded, the sample analysis system 100 acquires sample information of the sample under test to acquire sample information corresponding to the sample carried in the currently loaded sample vessel, the sample information at least includes a test item(s) of the sample, and then the sample analysis system 100 assigns a corresponding target analysis module to the loaded sample vessel, and tests the sample in the sample vessel through the target analysis module. The sample vessel may be loaded in at least one sample feeding mechanism of the sample analysis system, and the target analysis module configured to test the sample in the sample vessel includes at least one of the first analysis module 10 and the second analysis module 20. That is, the sample loaded from the first analysis module 10 may be dispatched to the second analysis module 20, so as to be tested, and the sample loaded from the second analysis module 20 may be dispatched to the first analysis module 10, so as to be tested.

Specifically, there may be several situations for assignment of the target analysis module configured to test the sample in the sample vessel, as follows.

First situation: when the sample vessel is loaded from the first sample feeding mechanism 121 of the first analysis module 10, and the sample analysis system 100 determines that the sample carried in the sample vessel needs to be analyzed by the first analysis module 10, the sample analysis system 100 dispatches the sample vessel from the first sample feeding mechanism 121 to the first test component 11 through the first dispatching mechanism 122, to test the sample vessel, thereby obtaining the first test result.

Second situation: when the sample vessel is loaded from the first sample feeding mechanism 121 of the first analysis module 10, and the sample analysis system 100 determines that the sample carried in the sample vessel needs to be analyzed by the second analysis module 20, the sample analysis system 100 dispatches the sample vessel from the first dispatching component 12 to the second analysis module 20 through the cascade dispatching mechanism 30, so as for the second test component 21to test the sample in the sample vessel.

Third situation: when the sample vessel is loaded from the first sample feeding mechanism 121 of the first analysis module 10, and the sample analysis system 100 determines that the sample carried in the sample vessel needs cascade test by the first analysis module 10 and the second analysis module 20, the sample analysis system 100 dispatches the sample vessel from the first sample feeding mechanism 121 to the first test component 11 through the first dispatching mechanism 122, so as to perform test to obtain the first test result.

After the first test component 11 completes aspiration or test of the sample, the first dispatching mechanism 122 dispatches the sample vessel back to a preset position of the first dispatching component 12, and the sample analysis system 100 dispatches the sample vessel from the first dispatching component 12 to the second analysis module 20 through the cascade dispatching mechanism 30, so as for the second test component 21 to test the sample in the sample vessel, thereby completing cascade test of the sample carried in the sample vessel.

Fourth situation: when the sample vessel is loaded from the second sample feeding mechanism 221 of the second analysis module 20, and the sample analysis system 100 determines that the sample carried in the sample vessel needs to be analyzed by the second analysis module 20, the sample analysis system 100 dispatches the sample vessel from the second sample feeding mechanism 221 to the second test component 21 through the second dispatching mechanism 222, so as to perform test to obtain the second test result.

Fifth situation: when the sample vessel is loaded from the second sample feeding mechanism 221 of the second analysis module 20, and the sample analysis system 100 determines that the sample carried in the sample vessel needs to be analyzed by the first analysis module 10, the sample analysis system 100 dispatches the sample vessel from the second dispatching component 22 to the first analysis module 10 through the cascade dispatching mechanism 30, so as for the first test component 11 to test the sample in the sample vessel.

Sixth situation: when the sample vessel is loaded from the second sample feeding mechanism 221 of the second analysis module 20, and the sample analysis system 100 determines that the sample carried in the sample vessel needs cascade test by the first analysis module 10 and the second analysis module 20, the sample analysis system 100 dispatches the sample vessel from the second sample feeding mechanism 221 to the second test component 21 through the second dispatching mechanism 222, so as to perform test to obtain the second test result.

After the second test component 21 completes aspiration or test of the sample, the second dispatching mechanism 222 dispatches the sample vessel back to a preset position of the second dispatching component 22, and the sample analysis system 100 dispatches the sample vessel from the second dispatching component 22 to the first analysis module 10 through the cascade dispatching mechanism 30, so as for the first test component 11 to test the sample in the sample vessel, thereby completing cascade test of the sample carried in the sample vessel.

It may be understood that the sample information corresponding to the sample in the sample vessel may be acquired as follows: the sample vessel is provided with an information code, and the information code of the sample vessel is scanned by a scanning mechanism arranged in the sample analysis system 100, to acquire the sample information of the sample carried by the sample vessel. The information code includes, but is not limited to, a bar code or a Quick Response (QR) code. Or, the user inputs corresponding sample information to the sample analysis system 100 by manipulating an information input device such as a mouse, a keyboard, a touch panel, a button, etc.

It may be understood that the scanning mechanism in the sample analysis system 100 may be the first scanning mechanism 123 arranged in the first dispatching component 12, or may be the second scanning mechanism 223 arranged in the second dispatching component 22, which is not limited here.

As may be seen from the above technical solution provided in the present application, the sample analysis system 100 provided in the present application is provided with the first analysis module 10 and the second analysis module 20, and each analysis module is provided with a sample feeding mechanism, a dispatching mechanism and a test mechanism, such that each analysis module may perform sample analysis independently through a respective dispatching mechanism. Furthermore, the sample analysis system 100 is further provided with the cascade dispatching mechanism 30, and the cascade dispatching mechanism 30 is configured to dispatch sample vessels between the first analysis module 10 and the second analysis module 20, such that the sample in each of the sample vessels loaded from the first analysis module 10 may enter the second analysis module 20 for test, or the sample in each of the sample vessels loaded from the second analysis module 20 may also enter the first analysis module 10 for test. Therefore, in case that the sample loaded from the first analysis module 10 needs to be tested in the second analysis module 20, manual dispatching is not needed, and accurate and efficient dispatching of the sample during test is further achieved, which further improves test efficiency of the sample and makes use experiences of the product better.

Furthermore, the cascade dispatching mechanism 30 is configured to dispatch sample vessels which need to be dispatched between the first analysis module 10 and the second analysis module 20, such that there is no need to additionally purchase a transit mechanism or a cascade sample feeding mechanism during cascade connection of the first analysis module 10 and the second analysis module 20.

In some implementations, the first analysis module 10 is detachably connected to the second analysis module 20, and the detachable connection may be implemented as follows: the first analysis module 10 further includes a first housing component 13, the first housing component 13 is formed with a first accommodation space, and the first dispatching component 12 and the first test component 11 are accommodated in the first accommodation space. The second analysis module 20 further includes a second housing component 23, the second housing component 23 is formed with a second accommodation space, and the second dispatching component 22 and the second test component 21 are accommodated in the second accommodation space. The first housing component 13 is provided with a first connection structure, the second housing component 23 is provided with a second connection structure, and the first connection structure is detachably connected to the second connection structure.

It may be understood that each of the first connection structure and the second connection structure may be a threaded connection structure or a snap connection structure, it merely needs to implement detachable connection between the first analysis module 10 and the second analysis module 20, and after the first analysis module 10 is detached and separated from the second analysis module 20, the first analysis module 10 and the second analysis module 20 can also perform sample analysis independently.

Further, in case that at least one of the first dispatching mechanism and the second dispatching mechanism is also used as the cascade dispatching mechanism 30, or in case that the cascade dispatching mechanism 30 is arranged in at least one of the first analysis module and the second analysis module, the first analysis module 10 and the second analysis module 20 may be put together to form a cascade instrument by connection components arranged on corresponding housings only, and there is no need to re-purchase a corresponding cascade dispatching mechanism, which may effectively save cost of connecting and cascade-connecting machines.

As shown in FIG. 2, in some implementations, the first sample feeding mechanism 121 is provided with multiple first sample feeding channels 1211 configured for the user to place one or more sample vessels that each sample vessel contains a sample. Optionally, multiple first sample feeding channels 1211 are arranged in a first horizontal direction and extend in a second horizontal direction, so as for the user to insert a sample holder into one of the first sample feeding channels 1211 along the second horizontal direction, and the sample holder is configured to hold the sample vessels.

As shown in FIG. 2, in some implementations, the second sample feeding mechanism 221 is provided with multiple second sample feeding channels 2211 configured for the user to place one or more sample vessels that each sample vessel contains a sample. Optionally, multiple second sample feeding channels 2211 are arranged in the first horizontal direction and extend in the second horizontal direction, so as for the user to insert a sample holder into one of the second sample feeding channels 2211 along the second horizontal direction, and the sample holder is configured to hold the sample vessels.

As shown in FIG. 2, in some implementations, in order to achieve efficient dispatching of sample vessels between the first analysis module 10 and the second analysis module 20, the first test component 11, the first dispatching component 12, the second dispatching component 22 and the second test component 21 are sequentially arranged in a first horizontal direction, and the first dispatching component 12 and the second dispatching component 22 are put together between the first test component 11 and the second test component 12.

With reference to FIG. 2, in some implementations, the first test component 11 includes a first test mechanism 111 and a first pipette mechanism 112. The first pipette mechanism 112 is configured to transfer the sample in each of the sample vessels on the first carrier mechanism 120 to the first test mechanism 111, so as for the first test mechanism 111 to perform test.

Exemplarily, when the target analysis module is the first analysis module 10, the first analysis module 10 may analyze the sample as follows: the first dispatching mechanism 122 or the cascade dispatching mechanism 30 dispatches sample vessels each carrying a sample to be analyzed to the first carrier mechanism 120, then the first pipette mechanism 112 transfers the sample in each of the sample vessels that are placed on the first carrier mechanism 120 to the first test mechanism 111, and the first test mechanism 111 tests the sample.

In some implementations, the second test component 21 includes a second test mechanism 211, a second pipette mechanism 212 and a second carrier mechanism 220. The second carrier mechanism 220 is configured to carry and support each of the sample vessels in which the sample is to be aspirated, and the second pipette mechanism 212 is configured to transfer the sample in each of the sample vessels on the second carrier mechanism 220 to the second test mechanism 211, so as for the second test mechanism 211 to perform test.

Exemplarily, when the target analysis module is the second analysis module 20, the second analysis module 20 may analyze the sample as follows: the second dispatching mechanism 222 or the cascade dispatching mechanism 30 dispatches sample vessels each carrying a sample to be analyzed to the second carrier mechanism 220, then the second pipette mechanism 212 transfers the sample in each of the sample vessels that are placed on the second carrier mechanism 220 to the second test mechanism 211, and the second test mechanism 211 performs test.

It may be understood that the first pipette mechanism 112 and/or the second pipette mechanism 212 includes a sample dispensing mechanism, a reagent member, and a reagent dispensing mechanism. The sample dispensing mechanism is configured to aspirate a sample and discharge the sample into a reaction vessel to be loaded(such as a reaction cup). For example, the sample dispensing mechanism may include a sample needle configured to perform two-dimensional (2D) or three-dimensional (3D) movement in space through a 2D or 3D driving mechanism, such that the sample needle can move to aspirate the sample carried by the carrier mechanism in the dispatching component, move to the reaction vessel to which the sample is to be loaded, and discharge the sample into the reaction vessel.

The reagent member is configured to carry a reagent. In an embodiment, the reagent member may be a reagent disk arranged in a disk-like structure and provided with multiple positions where reagent vessels are carried. The reagent member is rotatable and drives each of the reagent vessels carried by the reagent member to rotate, so as to make the reagent vessel rotated to a specific position, such as a position where the reagent is aspirated by the reagent dispensing mechanism. There may be one or more reagent members.

The reagent dispensing mechanism is configured to aspirate the reagent and discharge the reagent into the reaction vessel to which the reagent is to be loaded. In an embodiment, the reagent dispensing mechanism may include a sample needle configured to perform 2D or 3D movement in space through a 2D or 3D driving mechanism, such that the sample needle can move to aspirate the reagent carried by the reagent member, move to the reaction vessel to which the reagent is to be loaded, and discharge the reagent into the reaction vessel.

It may also be understood that the first test mechanism 111 and/or the second test mechanism 211 include a reaction member and an optical test member. The reaction member is provided with at least one arrangement position where the reaction vessel is arranged and a reaction solution in the reaction vessel is incubated. For example, the reaction member may be a reaction disk arranged in a disk-like structure and provided with one or more arrangement positions, at one of which the reaction vessel is arranged. The reaction disk is rotatable and drives the reaction vessel in its arrangement position to rotate, so as to dispatch the reaction vessel in the reaction disk and incubate the reaction solution in the reaction vessel.

The optical test member is configured to perform optical test on the incubated reaction solution, to obtain reaction data of the sample. For example, the optical test member tests a luminous intensity of a reaction solution under test, and calculates concentration of a component under test in the sample through a calibration curve. In an embodiment, the optical test member is separately arranged outside the reaction member.

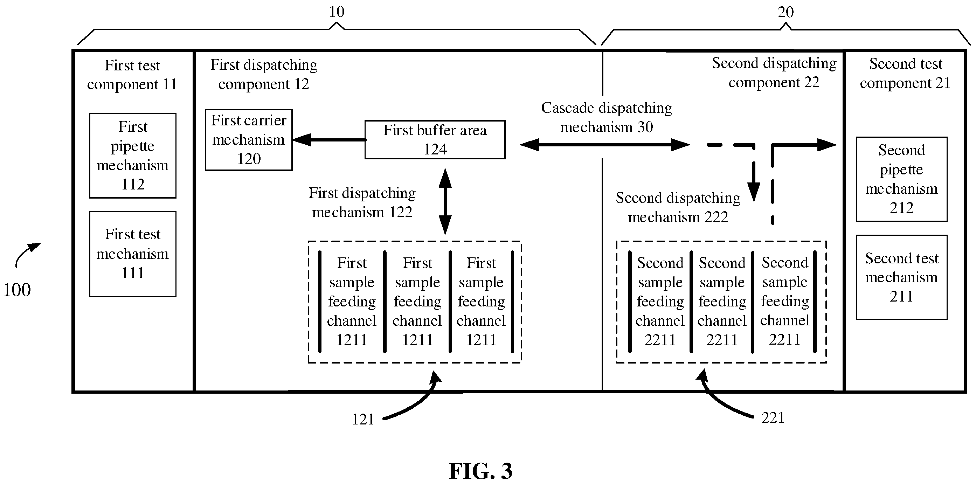

With reference to FIG. 3, in some implementations, a first buffer area 124 is provided in the first dispatching component 12, and the cascade dispatching mechanism 30 may dispatch sample vessels between the second dispatching component 22 and the first buffer area 124. The cascade dispatching mechanism 30 is configured to dispatch sample vessels provided by the second sample feeding mechanism 221 to the first buffer area 124, and the first dispatching mechanism 122 is configured to dispatch, in the first analysis module 10, the sample vessels in the first buffer area 124, so as for the first test component 11 to test at least a portion of the sample in each of at least a portion of the sample vessels provided by the second sample feeding mechanism 221 to obtain the first test result.

Exemplarily, when sample vessels are loaded by the second sample feeding mechanism 221 and the target analysis module is the first analysis module 10, the sample vessels may be dispatched from the second sample feeding mechanism 221 to the first buffer area 124 in the first dispatching component 12 by the cascade dispatching mechanism 30, and the sample vessels located in the first buffer area 124 may be dispatched by the first dispatching mechanism 122 to the first test component 11, so as for the first test component 11 to test at least a portion of the sample in each of the sample vessels provided by the second sample feeding mechanism 221 to obtain the first test result. For example, the first dispatching mechanism 122 dispatches the sample vessels that are loaded by the second sample feeding mechanism 221 in the first buffer area 124 to the first carrier mechanism 120, the first test component 11 transfers the sample in each of the sample vessels that are placed on the first carrier mechanism 120 to the first test mechanism 111 through the first pipette mechanism 112, and the first test mechanism 111 tests the sample.

As shown in FIG. 3, in some implementations, a first buffer area 124 is provided in the first dispatching component 12, the first dispatching mechanism 122 dispatches at least a portion of sample vessels in the first sample feeding mechanism 121 to the first buffer area 124, and the cascade dispatching mechanism 30 is configured to dispatch the sample vessels that are placed by the first dispatching component 12 in the first buffer area 124 to the second dispatching component 22, so as for the second test component 21 to perform test, to obtain the second test result.

Exemplarily, when sample vessels are loaded by the first sample feeding mechanism 121 and the target analysis module is the second analysis module 20, the sample vessels in the first sample feeding mechanism 121 may be dispatched by the first dispatching mechanism 122 to the first buffer area 124, and the cascade dispatching mechanism 30 is configured to dispatch the sample vessels in the first buffer area 124 to the second dispatching component 22 (for example, the cascade dispatching mechanism 30 dispatches the sample vessels to the second sample feeding mechanism 221 or the buffer area of the second dispatching component 22) to dispatch the sample vessels to the second test component 21 by the second dispatching mechanism 222 of the second dispatching component 22, so as for the second test component 21 to perform test to obtain the second test result.

In some implementations, at least one of the sample feeding channels 1211 is also used as the first buffer area 124, that is, at least one of the sample feeding channels 1211 is used as the first buffer area 124 to buffer the sample vessels.

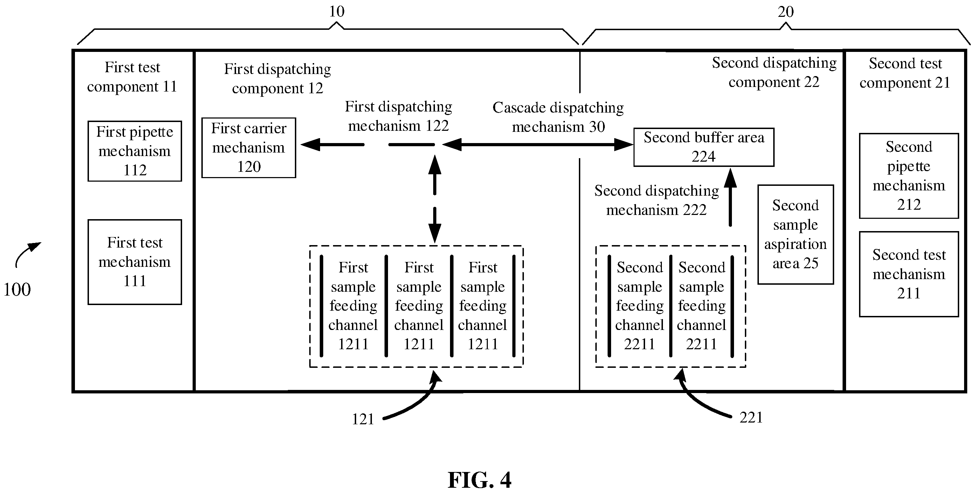

With reference to FIG. 4, in some implementations, a second buffer area 224 is provided in the second dispatching component 22, and the cascade dispatching mechanism 30 may dispatch sample vessels between the first dispatching component 12 and the second buffer area 224. The cascade dispatching mechanism 30 dispatches the sample vessels provided by the first sample feeding mechanism 121 to the second buffer area 224, and the second dispatching mechanism 222 is configured to dispatch, in the second analysis module 20, the sample vessels in the second buffer area 224, so as for the second test component 21 to test at least a portion of the sample in each of at least a portion of the sample vessels provided by the first sample feeding mechanism 121 to obtain the second test result.

Exemplarily, when sample vessels are loaded by the first sample feeding mechanism 121 and the target analysis module is the second analysis module 20, the sample vessels may be dispatched from the first sample feeding mechanism 121 to the second buffer area 224 in the second dispatching component 22 by the cascade dispatching mechanism 30, and the sample vessels located in the second buffer area 224 may be dispatched by the second dispatching mechanism 222 to the second test component 21, so as for the second test component 21 to test at least a portion of the sample in each of the sample vessels provided by the first sample feeding mechanism 121 to obtain the second test result. For example, the second dispatching mechanism 222 dispatches the sample vessels that are loaded by the first sample feeding mechanism 121 in the second buffer area 224 to the second carrier mechanism 220, the second test component 21 transfers the sample in each of the sample vessels that are placed on the second carrier mechanism 220 to the second test mechanism 211 through the second pipette mechanism 212, and the second test mechanism 211 tests the sample.

In some implementations, a second buffer area 224 is provided in the second dispatching component 22, the second dispatching mechanism 222 dispatches at least a portion of sample vessels in the second sample feeding mechanism 221 to the second buffer area 224, and the cascade dispatching mechanism 30 is configured to dispatch the sample vessels that are placed by the second dispatching component 22 in the second buffer area 224 to the first dispatching component 12, so as for the first test component 11 to perform test, to obtain the first test result.

Similarly, when sample vessels are loaded by the second sample feeding mechanism 221 and the target analysis module is the first analysis module 10, the sample vessels in the second sample feeding mechanism 221 may be dispatched by the second dispatching mechanism 222 to the second buffer area 224, and the cascade dispatching mechanism 30 is configured to dispatch the sample vessels in the second buffer area 224 to the first dispatching component 12 (for example, the cascade dispatching mechanism 30 dispatches the sample vessels to the first sample feeding mechanism 121 or the first buffer area of the first dispatching component 12) to dispatch the sample vessels to the first test component 11 by the first dispatching mechanism 122 of the first dispatching component 12, so as for the first test component 11 to perform test to obtain the first test result.

In some implementations, the second analysis module 20 is provided with a second sample aspiration area 25, and the cascade dispatching mechanism 30 may dispatch the sample vessels between the first dispatching component 12 and the second buffer area 224. The cascade dispatching mechanism 30 dispatches the sample vessels provided by the first sample feeding mechanism 121 to the second buffer area 224, and the second dispatching mechanism 222 dispatches the sample vessels in the second buffer area 224 to the second sample aspiration area, so as for the second test component 21 to test at least a portion of the sample in each of at least a portion of the sample vessels provided by the first sample feeding mechanism 121 to obtain the second test result.

In the implementation corresponding to FIG. 3 and FIG. 4, a buffer area is provided in at least one of the first analysis module 10 and the second analysis module 20, and the sample analysis system is provided with the cascade dispatching mechanism 30, such that after the first analysis module 10 is cascade-connected with the second analysis module 20, the sample vessels which need to be dispatched between the first analysis module 10 and the second analysis module 20 are buffered and transited by using the buffer area, which facilitates cascade dispatching of the sample analysis system. Furthermore, there is no need to additionally purchase a transit mechanism or a cascade sample feeding mechanism during cascade connection of the first analysis module 10 and the second analysis module 20.

Furthermore, the cascade dispatching mechanism 30 is provided, such that in case that it needs to dispatch sample vessels between the first analysis module 10 and the second analysis module 20 to test a sample(s) in a corresponding target analysis module, manual dispatching is not needed, and accurate and efficient dispatching of the sample(s) during test is further achieved, which further improves test efficiency of the sample and makes use experiences of the product better

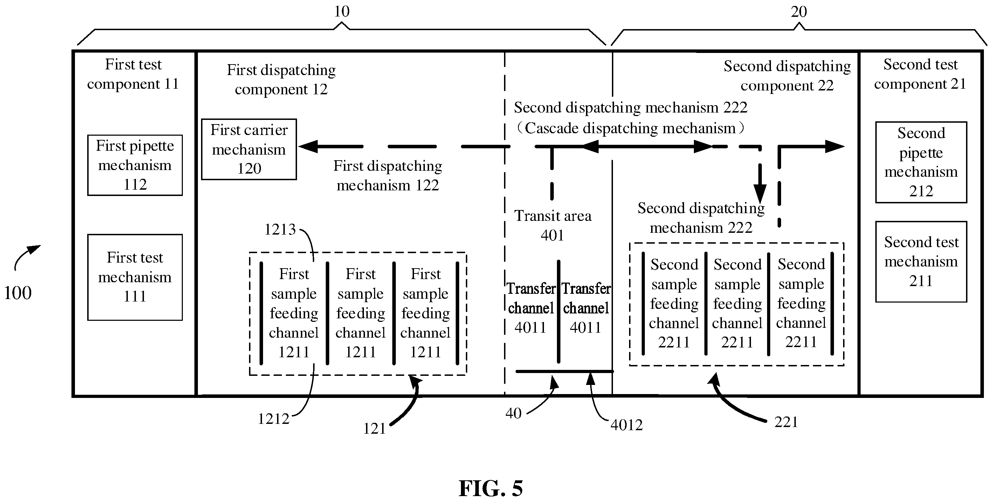

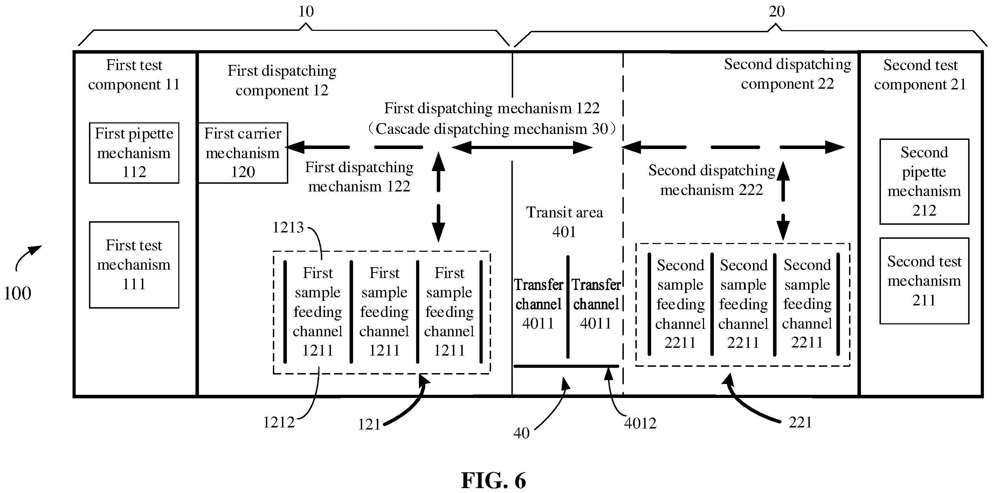

With reference to FIG. 5 and FIG. 6, in some implementations, at least one of the first dispatching mechanism 122 and the second dispatching mechanism 222 is also used as the cascade dispatching mechanism 30. The sample analysis system 100 further includes a transit mechanism 40 formed with a transit area 401, the second dispatching mechanism 222 dispatches sample vessels between the transit area 401 and the second dispatching component 22, and the first dispatching mechanism 122 dispatches sample vessels between the transit area 401 and the first dispatching component 12. The first dispatching mechanism 122 is further configured to dispatch at least a portion of the sample vessels provided by the first sample feeding mechanism 121 to the transit area 401, and the second dispatching mechanism 222 is further configured to dispatch the sample vessels in the transit area 401 to the second analysis module 20, so as for the second test component 21 to test at least a portion of the sample in each of the sample vessels dispatched by the second dispatching mechanism 222 to obtain the second test result.

It may be understood that the transit mechanism 40 may be arranged independently of the first analysis module 10 and the second analysis module 20, or may be arranged in at least one of the first analysis module 10 and the second analysis module 20, which is not limited here.

Optionally, as shown in FIG. 5, the transit mechanism 40 is arranged in the first analysis module, and at least the second dispatching mechanism 222 is also used as the cascade dispatching mechanism.

Optionally, as shown in FIG. 6, the transit mechanism 40 is arranged in the second analysis module, and at least the first dispatching mechanism is also used as the cascade dispatching mechanism.

Optionally, the transit mechanism 40 is arranged in the first dispatching component, and at least the second dispatching mechanism 222 is also used as the cascade dispatching mechanism. Or, the transit mechanism 40 is arranged in the second dispatching component 22, and at least the first dispatching mechanism is also used as the cascade dispatching mechanism.

As shown in FIG. 5, descriptions are given by taking an example that the transit mechanism 40 is arranged in the first analysis module and the second dispatching mechanism 122 is also used as the cascade dispatching mechanism 30.

When sample vessels are loaded by the first sample feeding mechanism 121 and the target analysis module is the second analysis module 20, the sample vessels are dispatched by the first dispatching component 122 to the transit area 401 of the transit mechanism 40, then the sample vessels in the transit area 401 are dispatched by the second dispatching mechanism 222 (i.e., the cascade dispatching mechanism 30) to the second analysis module 20, and the sample vessels are dispatched in the second analysis module 20 by the second dispatching mechanism 222, so as for the second test component 21 to test at least a portion of the sample in each of the sample vessels dispatched by the second dispatching mechanism 222 to obtain the second test result.

When sample vessels are loaded by the second sample feeding mechanism 221 and the target analysis module is the first analysis module 10, the sample vessels are dispatched by the second dispatching component 122 (the cascade dispatching mechanism 30) to the transit area 401 of the transit mechanism 40, then the sample vessels in the transit area 401 are dispatched by the first dispatching mechanism 122 to the first analysis module 10, and the sample vessels are dispatched in the first analysis module 10 by the first dispatching mechanism 122, so as for the first test component 11 to test at least a portion of the sample in each of the sample vessels dispatched by the first dispatching mechanism 122 to obtain the first test result.

In the implementation corresponding to FIG. 5 and FIG. 6, the transit mechanism formed with the transit area is provided in at least one of the first analysis module 10 and the second analysis module 20, and the dispatching mechanism of the first analysis module 10 or the dispatching mechanism of the second analysis module 20 is also used as the cascade dispatching mechanism, such that after the first analysis module 10 is cascade-connected with the second analysis module 20, the sample vessels which need to be dispatched between the first analysis module 10 and the second analysis module 20 are buffered and transited by using the transit area, which facilitates cascade dispatching of the sample analysis system. Furthermore, there is no need to additionally purchase a transit mechanism or a cascade sample feeding mechanism during cascade connection of the first analysis module 10 and the second analysis module 20.

Furthermore, the dispatching mechanism of the first analysis module 10 or the dispatching mechanism of the second analysis module 20 is also used as the cascade dispatching mechanism, such that in case that it needs to dispatch sample vessels between the first analysis module 10 and the second analysis module 20 to test a sample(s) in a corresponding target analysis module, manual dispatching is not needed, and accurate and efficient dispatching of the sample(s) during test is further achieved, which further improves test efficiency of the sample and makes use experiences of the product better.

With reference to FIG. 5, in some implementations, the transit mechanism 40 is arranged in the first dispatching component 12, transfer channels 4011 extending in the second horizontal direction are provided in the transit area 401 of the transit mechanism 40 to accommodate the sample vessels placed by the second dispatching mechanism 222. The transfer channels 4011 and the first sample feeding channels 1211 are arranged in a first direction. The transfer channels 4011 are located at a side of the first dispatching component 12 close to the second dispatching component 22, and the second horizontal direction is perpendicular to the first horizontal direction.

In some implementations, the transit mechanism 40 is located in the first sample feeding mechanism 121, and at least one of the first sample feeding channels 1211 is also used as the transfer channel 4011. For example, in case that the first sample feeding channels 1211 are idle, at least one of the first sample feeding channels 1211 is used as the transfer channel 4011.

With reference to FIG. 6, in some implementations, the transit mechanism 40 is arranged in the second dispatching component 22, a transfer channel(s) 4011 extending in the second horizontal direction are provided in the transit area 401 of the transit mechanism 40 to accommodate the sample vessels that are placed by the first dispatching mechanism 122. The transfer channel(s) 4011 and the second sample feeding channels 2211 are arranged in a first direction. The transfer channel(s) 4011 are located at a side of the second dispatching component 22 close to the first dispatching component 12. The second horizontal direction is perpendicular to the first horizontal direction.

Preferably, the transit mechanism 40 is arranged in the first dispatching component 12, and the transfer channel(s) 4011 are located at a side of the second dispatching component 22 close to the first dispatching component 12.

In some implementations, the transit mechanism 40 is located in the second sample feeding mechanism 221, and at least one of the second sample feeding channels 2211 is also used as the transfer channel(s) 4011. For example, in case that the second sample feeding channels 2211 are idle, at least one of the second sample feeding channels 2211 is used as the transfer channel(s) 4011.

In some implementations, the first dispatching component 12 includes a first side and a second side in the second horizontal direction. The first sample feeding channel 1211 includes a sample vessel inlet 1212 close to the first side and a sample vessel outlet 1213 close to the second side. A baffle 4012 is arranged at an end of the transfer channel(s) 4011 close to the first side, so as to shield an opening of the transfer channel(s) 4011 close to the first side.

In the implementation, openings of both the transfer channel(s) 4011 and the first sample feeding channel(s) 1211 face the same direction. In some cases, the user may use the transfer channel 4011(s) as a sample feeding channel(s), and thus may place a non-cascade-connected sample(s) in the transfer channel(s) 4011, thereby occupying the transfer channel(s) 4011 and affecting test efficiency for sample analysis. In the implementation, the baffle 4012 is arranged at the opening(s) of the transfer channel(s) 4011, and thus can effectively prevent the user from placing a sample vessel(s) in the transfer channel(s).

Optionally, when a first sample feeding channel 1211 is also used as a transfer channel 4011, the baffle 4012 is a movable baffle, which is switchable between a first state and a second state. In case that no first sample feeding channel 1211 is also used as the transfer channel 4011, the baffle 4012 is in the second state, in which the baffle 4012 is retracted toward a side away from the opening to expose the opening; and at this time, the baffle 4012 does not shield the opening of the first sample feeding channel 1211, such that the user may place the sample vessels in the first sample feeding channel 1211. In case that a first sample feeding channel 1211 is also used as a transfer channel 4011, the baffle 4012 is in the first state, in which the baffle 4012 extends toward the opening, so as to shield the opening.

As shown in FIG. 1, FIG. 5 and FIG. 6, in some implementations, the sample analysis system 100 further includes a controller 50, the controller 50 may be one or more controllers, and the controller 50 may be arranged in at least one of the first analysis module 10 and the second analysis module 20, or may be arranged independently of the first analysis module 10 and the second analysis module 20, which is not limited here.

In some implementations, the transit mechanism 40 is arranged in the first dispatching component 12, and the controller 50 is configured to, in response to detecting that a sample vessel(s) is/are placed by the user in the transfer channel(s) 4011, control the first dispatching mechanism 122 to dispatch the sample vessel(s) in the transfer channel 4011 to a first buffer area 124 in the first dispatching component 12; or, control an alarm device 60 of the sample analysis system 100 to output a first alarm information, to inform the user of an abnormal situation by using the first alarm information, for example, the transfer channel 4011 is occupied.

And/or, the controller 50 is further configured to, in response to detecting that a sample vessel(s) that is/are placed in the transfer channel 4011 is/are removed by the user, control the alarm device 60 of the sample analysis system 100 to output a second alarm information, to inform the user of an abnormal situation by using the second alarm information, for example, the sample vessel(s) in the transfer channel 4011 is/are removed by the user.

Or, the transit mechanism 40 is arranged in the second dispatching component 22, and the controller 50 is configured to control the second dispatching mechanism 222 to dispatch the sample vessels in the transfer channel 4011 to a second buffer area 224 in the second dispatching component 22, or control the alarm device 60 of the sample analysis system 100 to output the first alarm information, in response to detecting that the sample vessel(s) is/are placed by the user in the transfer channel 4011.

It may be understood that manner of determining whether a sample vessel(s) is/are placed by the user in the transfer channel 4011 or a sample vessel(s) is/are removed by the user from the transfer channel 4011 may be determining that a sample vessel(s) is/are placed by the user in the transfer channel 4011, when it is detected that the sample vessel(s) is/are placed in the transfer channel 4011, the first dispatching mechanism 122 and the second dispatching mechanism 222 do not place a sample vessel(s) in the transfer channel 4011, and it is detected that the sample vessel(s) is/are in the transfer channel 4011.

Or, it is determined that a sample vessel(s) is/are removed by the user from the transfer channel 4011, when it is detected that the sample vessel(s) is/are removed from the transfer channel 4011, neither of the first dispatching mechanism 122 and the second dispatching mechanism 222 removes the sample vessel(s) from the transfer channel, and the sample vessel(s) is/are not in the transfer channel 4011.

Exemplarily, the alarm device 60 includes, but is not limited to at least one of a buzzer, a display, and an indicator light. Usually, the transfer channel 4011 is configured to store sample vessels which need to be transferred (such as cascade-connected sample vessels, or sample vessels which are not tested by the system). In order to ensure safety during transfer of the sample vessels, the sample analysis system 100 is provided with a sensing device configured to monitor operation states of the sample analysis system 100, for example, the sensing device is at least configured to detect whether the sample vessels are placed in the transfer channel 4011, and detect dispatching operation states of the first dispatching mechanism 122 and the second dispatching mechanism 222. The dispatching operation state includes at least whether the dispatching mechanism performs a dispatching operation, time of performing the dispatching operation, a dispatching object of the dispatching operation.

When the sensing device tests that a sample vessel(s) is/are placed by the user in the transfer channel 4011, the sensing device sends corresponding first feedback information to the controller 50, such that the controller 50 controls the dispatching mechanism of the analysis module where the transfer channel 4011 is located to remove the sample vessel(s) from the transfer channel 4011 according to the received first feedback information, to avoid the transfer channel 4011 from being occupied for a long time and affecting cascade dispatching of the sample vessels. Or, the sensing device outputs corresponding alarm information, to prompt the user to remove abnormal sample vessel(s) from the transfer channel in time.

Similarly, when the sensing device detects that the sample vessel(s) that is/are placed in the transfer channel 4011 is/are removed by the user, the sensing device outputs corresponding alarm information, to prompt the user that the sample vessel(s) in the transfer channel is/are removed abnormally.

In some implementations, during start-up of the sample analysis system 100, a transit prompt information is displayed on a preset display interface of at least one of the first analysis module 10 and the second analysis module 20, and is configured to prompt the user not to remove the sample vessel(s) from the transfer channel 4011, and/or configured to prompt the user not to place sample vessel(s) in the transfer channel 4011.

In some implementations, the sample analysis system 100 further includes a display, and the controller 50 is configured to control the display to display a user interaction interface including a first display area and a second display area. A schematic arrangement diagram of the first sample feeding channels 1211 and the transfer channels 4011 is displayed in the first display area, a schematic arrangement diagram of the first sample feeding channels 1211 and the transfer channels 4011 is displayed in the second display area, the first display area and the second display area do not overlap at least partially, and display parameters of the transfer channel 4011 are different from those of the first sample feeding channel 1211. The display parameters include, but are not limited to color, shape and position.

It may be understood that the display may be arranged in the first analysis module 10, or may be arranged in the second analysis module 20, or may be arranged independently of the first analysis module 10 and the second analysis module 20, which is not limited here.

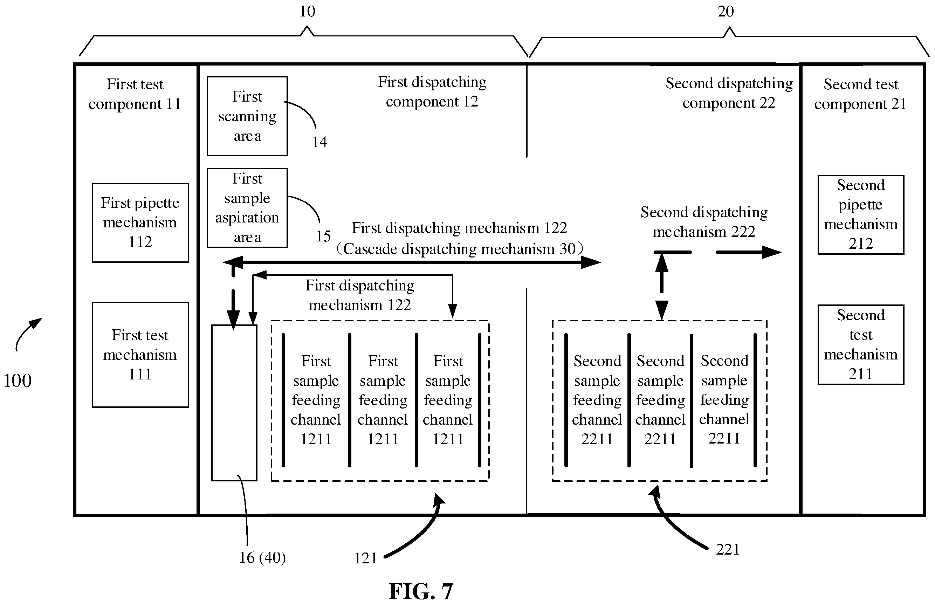

With reference to FIG. 1 and FIG. 7, in some implementations, the first analysis module 10 further includes a first scanning mechanism 123. The first dispatching component 12 is further provided with a first scanning area 14 and a first sample aspiration area 15. The first scanning mechanism 123 is configured to scan sample vessels in the first scanning area 14 to acquire sample information of the sample carried by each of the sample vessels, the first pipette mechanism 112 is configured to transfer at least a portion of the sample in each of sample vessels in the first sample aspiration area 15 to the first test mechanism 111, such that the first test mechanism 111 tests the sample to obtain a test result. The first dispatching mechanism 122 is further configured to transfer at least a portion of the sample vessels provided by the first sample feeding mechanism 121 to the first scanning area 14 to scan at least a portion of the sample vessels, and dispatch at least a portion of the scanned sample vessels to the first sample aspiration area 15.

As shown in FIG. 7, in some implementations, the first dispatching component 12 is provided with a first test buffer area 16, and the first dispatching mechanism 122 is further configured to transfer at least a portion of the scanned sample vessels to the first test buffer area 16 to buffer at least a portion of the scanned sample vessels, and dispatch the sample vessels buffered in the first test buffer area 16 to the first sample aspiration area 15. The transit mechanism 40 is located in the first test buffer area 16, or the transit mechanism 40 is located in the first scanning area 14.

In the implementation corresponding to FIG. 7, the test buffer area or the scanning area provided in the sample analysis system 100 is also used as the transit area, and the dispatching mechanism of the first analysis module 10 or the dispatching mechanism of the second analysis module 20 is also used as the cascade dispatching mechanism, such that after the first analysis module 10 is cascade-connected with the second analysis module 20, the sample vessels which need to be dispatched between the first analysis module 10 and the second analysis module 20 are buffered and transited by using the transit area, which facilitates cascade dispatching of the sample analysis system. Furthermore, there is no need to additionally purchase a transit mechanism or a cascade sample feeding mechanism during cascade connection of the first analysis module 10 and the second analysis module 20 .

Furthermore, the dispatching mechanism of the first analysis module 10 or the dispatching mechanism of the second analysis module 20 is also used as the cascade dispatching mechanism, such that in case that it needs to dispatch sample vessels between the first analysis module 10 and the second analysis module 20 to test a sample(s) in a corresponding target analysis module, manual dispatching is not needed, and accurate and efficient dispatching of the sample(s) during test is further achieved, which further improves test efficiency of the sample and makes use experiences of the product better.

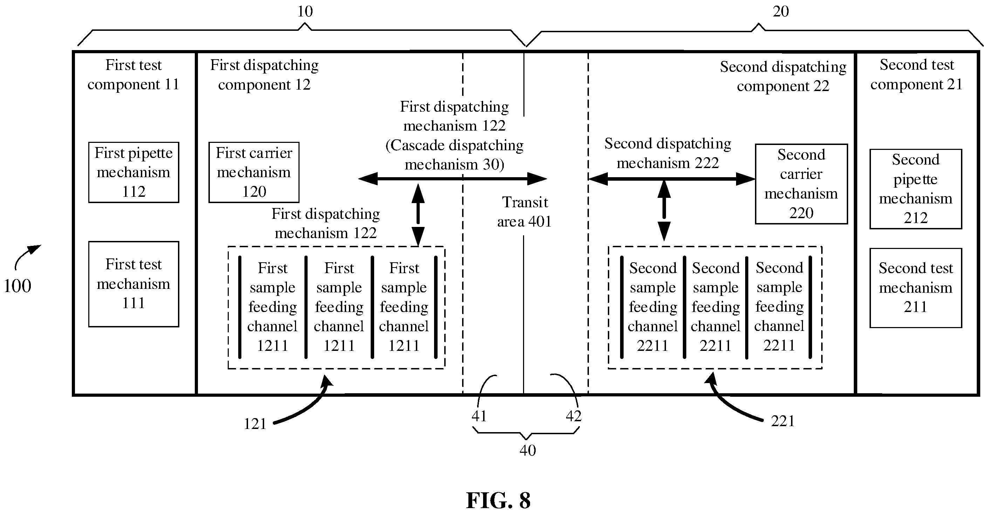

With reference to FIG. 8, in some implementations, at least one of the first dispatching mechanism 122 and the second dispatching mechanism 222 is also used as the cascade dispatching mechanism 30, the first analysis module 10 is provided with a first transit part 41, and the second analysis module 20 is provided with a second transit part 42. The first transit part 41 is arranged in the first dispatching component 12, the second transit part 42 is arranged in the second dispatching component 22, and the first transit part 41 and the second transit part 42 are put together to form a transit mechanism 40 with a transit area 401. The second dispatching mechanism 222 dispatches sample vessels between the transit area 401 and the second dispatching component 22, and the first dispatching mechanism 122 dispatches sample vessels between the transit area 401 and the first dispatching component 12. The first dispatching mechanism 122 is further configured to dispatch at least a portion of the sample vessels provided by the first sample feeding mechanism 121 to the transit area 401, and the second dispatching mechanism 222 is further configured to dispatch sample vessels in the transit area 401 to the second analysis module 20, so as for the second test component 21 to test at least a portion of the sample in each of the sample vessels dispatched by the second dispatching mechanism 222, to obtain the second test result. Or, the second dispatching mechanism 222 is further configured to dispatch at least a portion of the sample vessels provided by the second sample feeding mechanism 221 to the transit area 401, and the first dispatching mechanism 122 is further configured to dispatch the sample vessels in the transit area 401 to the first analysis module 10, so as for the first test component 11 to test at least a portion of the sample in each of the sample vessels dispatched by the first dispatching mechanism 122, to obtain the first test result.

In the implementation corresponding to FIG. 8, at least one of the first dispatching mechanism 122 and the second dispatching mechanism 222 is also used as the cascade dispatching mechanism 30, and each analysis module is provided with a respective transit part. The first transit part 41 of the first analysis module 10 is arranged close to the second analysis module 20, and the second transit part 42 of the second analysis module 20 is arranged close to the first analysis module 10. After the first analysis module 10 is cascade-connected with the second analysis module 20, the first transit part 41 and the second transit part 42 are put together to form the transit mechanism 40 with the transit area 401, the sample vessels which need to be dispatched between the first analysis module 10 and the second analysis module 20 are buffered and transited by using the transit area 401, which facilitates cascade dispatching of the sample analysis system. Furthermore, there is no need to additionally purchase a transit mechanism or a cascade sample feeding mechanism during cascade connection of the first analysis module 10 and the second analysis module 20.

Furthermore, the dispatching mechanism of the first analysis module 10 or the dispatching mechanism of the second analysis module 20 is also used as the cascade dispatching mechanism, such that in case that it needs to dispatch sample vessels between the first analysis module 10 and the second analysis module 20 to test a sample(s) in a corresponding target analysis module, manual dispatching is not needed, and accurate and efficient dispatching of the sample(s) during test is further achieved, which further improves test efficiency of the sample and makes use experiences of the product better.

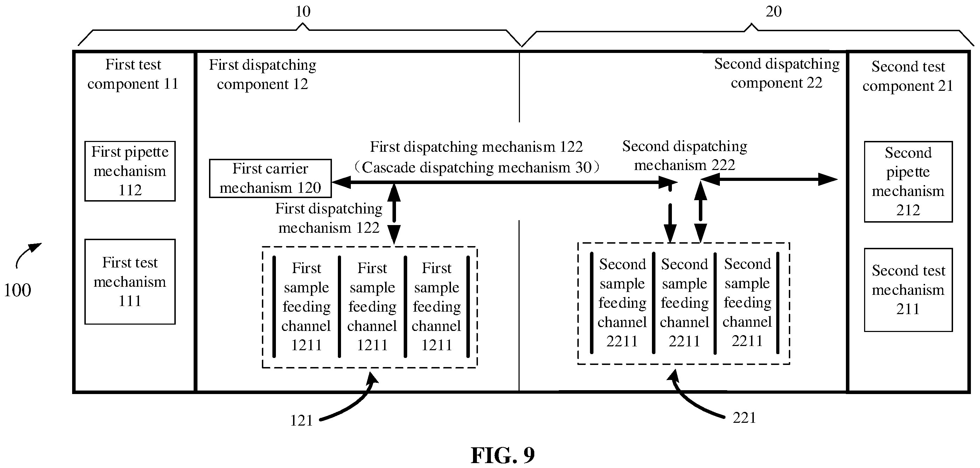

With reference to FIG. 9, in some implementations, the first sample feeding mechanism 121 is provided with multiple first sample feeding channels 1211, the second sample feeding mechanism 221 includes multiple second sample feeding channels 2211, at least the second dispatching mechanism 222 is also used as the cascade dispatching mechanism 30, and a dispatching trajectory of the second dispatching mechanism 222 may reach the first sample feeding channel 1211 and the second sample feeding channel 2211. The second dispatching mechanism 222 is further configured to dispatch at least a portion of sample vessels in the first sample feeding channel 1211 to the second analysis module 20, so as for the second test component 21 to test at least a portion of the sample in each of the sample vessels dispatched by the second dispatching mechanism 222, to obtain the second test result.

In some implementations, the second sample feeding mechanism 221 is provided with multiple second sample feeding channels 1211, at least the first dispatching mechanism 122 is also used as the cascade dispatching mechanism 30, and the first dispatching mechanism 122 is further configured to dispatch sample vessels in the second sample feeding channel 1211 to the first analysis module 10, so as for the first test component 11 to test at least a portion of the sample in each of the sample vessels dispatched by the first dispatching mechanism 122, to obtain the first test result.

In the implementation corresponding to FIG. 9, at least one of the first dispatching mechanism 122 and the second dispatching mechanism 222 is also used as the cascade dispatching mechanism 30, and the cascade dispatching mechanism 30 may dispatch sample vessels in the analysis module where the cascade dispatching mechanism 30 is located, or may dispatch sample vessels between the sample feeding channel in another analysis module and the analysis module where the cascade dispatching mechanism 30 is located. The sample vessels which need to be dispatched between two analysis modules are buffered by using a corresponding sample feeding channel, and therefore there is no need to additionally purchase a transit mechanism or cascade sample feeding mechanisms during cascade connection of the first analysis module 10 and the second analysis module 20 of the sample analysis system to achieve cascade dispatching.

Furthermore, the dispatching mechanism of the first analysis module 10 or the dispatching mechanism of the second analysis module 20 is also used as the cascade dispatching mechanism, such that in case that it needs to dispatch sample vessels between the first analysis module 10 and the second analysis module 20 to test a sample(s) in a corresponding target analysis module, manual dispatching is not needed, and accurate and efficient dispatching of the sample(s) during test is further achieved, which further improves test efficiency of the sample and makes use experiences of the product better.

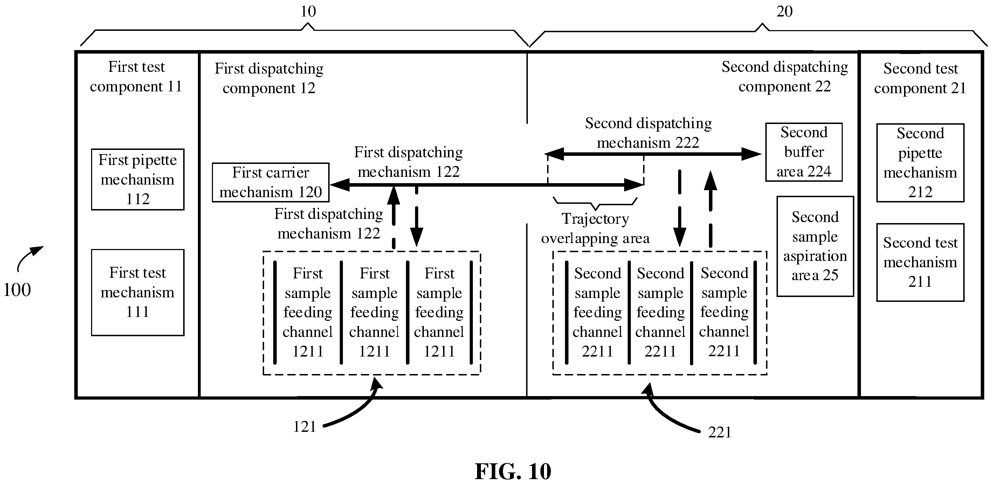

With reference to FIG. 10, in some implementations, at least one of the first dispatching mechanism 122 and the second dispatching mechanism 222 is also used as the cascade dispatching mechanism 30, dispatching trajectories of the first dispatching mechanism 122 and the second dispatching mechanism 222 at least partially overlap with each other, the first dispatching mechanism 122 is further configured to transfer at least a portion of the sample vessels provided by the first sample feeding mechanism 121 to the second dispatching mechanism 222, and the second dispatching mechanism 222 is further configured to transfer, in the second analysis module 20, the sample vessels received from the first dispatching mechanism 20.

Exemplarily, dispatching trajectories of the first dispatching mechanism 122 and the second dispatching mechanism 222 partially overlap with each other, such that the first dispatching mechanism 122 may receive sample vessels dispatched by the second dispatching mechanism 222 from the second sample feeding mechanism 221, or the second dispatching mechanism 222 may receive sample vessels dispatched by the first dispatching mechanism 122 from the first sample feeding mechanism 121.

Therefore, in case that at least one of the first dispatching mechanism 122 and the second dispatching mechanism 222 is also used as the cascade dispatching mechanism 30, dispatching trajectories of the first dispatching mechanism 122 and the second dispatching mechanism 222 partially overlap with each other. Accordingly, there is no need to additionally purchase a transit mechanism or a cascade sample feeding mechanism during cascade connection of the first analysis module 10 and the second analysis module 20 of the sample analysis system to achieve cascade dispatching..

Furthermore, the dispatching mechanism of the first analysis module 10 or the dispatching mechanism of the second analysis module 20 is also used as the cascade dispatching mechanism. Therefore, in case that it needs to dispatch sample vessels between the first analysis module 10 and the second analysis module 20 to test a sample(s) in a corresponding target analysis module, manual dispatching is not needed, and accurate and efficient dispatching of the sample during test is further achieved, which further improves test efficiency of the sample and makes use experiences of the product better.

Optionally, when both the first dispatching mechanism 122 and the second dispatching mechanism 222 are dispatching trolleys, dispatching of the sample vessels between the first dispatching mechanism 122 and the second dispatching mechanism may be performed in the following manner: the first dispatching mechanism 122 includes a first dispatching trolley, and the first dispatching trolley is provided with a first carrying position(s); the second dispatching mechanism 222 includes a second dispatching trolley, and the second dispatching trolley is provided with a second carrying position(s); the first dispatching trolley and/or the second dispatching trolley are provided with a transfer component, the first dispatching trolley and the second dispatching trolley are aligned to form a transfer channel in a process of transferring at least a portion of the sample vessels loaded by the second sample feeding mechanism to the first dispatching mechanism, and the transfer component applies force to the sample vessel(s) located at the second carrying position(s), to transfer the sample vessel(s) carried at the second carrying position(s) to the first carrying position(s) through the transfer channel.

Optionally, when both the first dispatching mechanism 122 and the second dispatching mechanism 222 are mechanical arms, dispatching of the sample vessels between the first dispatching mechanism 122 and the second dispatching mechanism may be performed in the following manner: the first dispatching mechanism includes a first mechanical gripper, the second dispatching mechanism includes a second mechanical gripper, and the sample vessel(s) is/are carried in the sample holder; in a process of transferring at least a portion of the sample vessels loaded by the second sample feeding mechanism to the first dispatching mechanism, the second mechanical gripper performs a grasp action at a second position of the sample holder and transfers the sample holder to a grasping range of the first mechanical gripper, such that the first mechanical gripper performs a grasp action at a first position different from the second position of the sample holder.

Optionally, when both the first dispatching mechanism 122 and the second dispatching mechanism 222 are conveyor belts, the first dispatching mechanism includes a first conveyor belt, and the second dispatching mechanism includes a second conveyor belt; in a process of transferring at least a portion of the sample vessels loaded by the second sample feeding mechanism to the first dispatching mechanism, the second conveyor belt is aligned with the first conveyor belt, and transfers the sample vessels to the first conveyor belt.

As shown in FIG. 10, in some implementations, the second analysis module 20 is provided with a second buffer area 224 and a second sample aspiration area 25. The second dispatching mechanism 222 is configured to dispatch sample vessels received from the first dispatching mechanism 122 to the second buffer area 224, and the second dispatching mechanism 222 is further configured to dispatch sample vessels in the second buffer area 224 to the second sample aspiration area 25, so as for the second test component 21 to test at least a portion of the sample in each of the sample vessels located in the second sample aspiration area 25, to obtain the second test result.

Optionally, the second sample feeding mechanism 221 is provided with multiple second sample feeding channels 2211, and at least one of the second sample feeding channels 2211 is also used as the second buffer area 224.

As shown in FIG. 10, in some implementations, the second analysis module 10 further includes a second pipette mechanism 212 and a second test mechanism 211, the second dispatching component 22 is provided with a second sample aspiration area 25, the second pipette mechanism 212 is configured to transfer at least a portion of the sample in each of sample vessels in the second sample aspiration area 25 to the second test mechanism 211, such that the second test mechanism 211 tests at least a portion of the sample in each of the sample vessels to obtain a test result, and the second dispatching mechanism 222 dispatches sample vessels received from the first dispatching mechanism 122 to the second sample aspiration area 25.

As shown in FIG. 1 and FIG. 10, in some implementations, the second dispatching component 22 is provided with a second buffer area 224, and the sample analysis system 100 further includes a controller 50 configured to, before the second dispatching mechanism 222 dispatches the sample vessels received from the first dispatching mechanism 122 to the second sample aspiration area 25:

determine whether the second sample aspiration area 25 meets a sample vessel arrangement condition, and

control the second dispatching mechanism 222 to dispatch the sample vessels received from the first dispatching mechanism 122 to the second sample aspiration area 25, in response to the second sample aspiration area 25 meeting the sample vessel arrangement condition; and control the second dispatching mechanism 222 to dispatch the sample vessels received from the first dispatching mechanism 122 to the second buffer area 224, in response to the second sample aspiration area 25 not meeting the sample vessel arrangement condition.

It may be understood that determining whether the second sample aspiration area 25 meets the sample vessel arrangement condition may be: determining whether there is at least one idle sample aspiration position in the second sample aspiration area 25; if yes, indicating that the second sample aspiration area 25 meets the sample vessel arrangement condition; otherwise, indicating that the second sample aspiration area 25 does not meet the sample vessel arrangement condition.

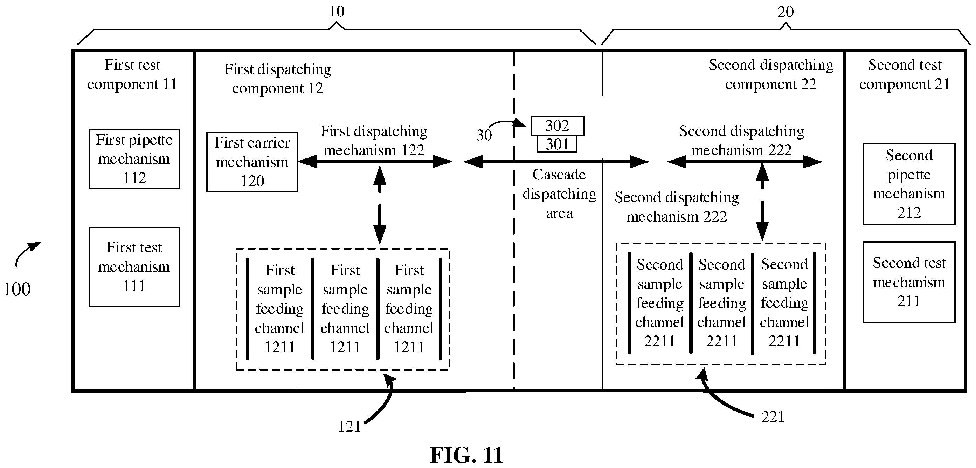

With reference to FIG. 11, in some implementations, the cascade dispatching mechanism 30 is arranged in the first dispatching component 12 and/or the second dispatching component 22, and the cascade dispatching mechanism 30, the second dispatching mechanism 222 and the first dispatching mechanism 122 may dispatch sample vessels independently of one another.

In some implementations, the first dispatching component 12 and the second dispatching component 22 are put together in a first horizontal direction. The cascade dispatching mechanism 30 includes a drive member 301 and a transfer member 302 configured to carry the sample vessels. The drive member 301 is configured to drive the transfer member 302 to move between the first dispatching component 12 and the second dispatching component 22 in the first horizontal direction. The first dispatching mechanism 122 is configured to dispatch, in the first analysis module 10, sample vessels carried on the transfer member 302 in the first dispatching component 12. The second dispatching mechanism 222 is configured to dispatch, in the second analysis module 20, sample vessels carried on the transfer member 302 in the second dispatching component 22.

In the implementation, an independent cascade dispatching mechanism 30 is arranged in at least one of the first analysis module 10 and the second analysis module 20, and the cascade dispatching mechanism 30 moves between the first dispatching component 12 and the second dispatching component 22 in the first horizontal direction, thereby achieving dispatching of sample vessels between the first analysis module 10 and the second analysis module 20. Accordingly, there is no need to additionally purchase a transit mechanism or a cascade sample feeding mechanism during cascade connection of the first analysis module 10 and the second analysis module 20 of the sample analysis system to achieve cascade dispatching.

Furthermore, the dispatching mechanism of the first analysis module 10 or the second analysis module 20 is also used as the cascade dispatching mechanism. Therefore, in case that it needs to dispatch sample vessels between the first analysis module 10 and the second analysis module 20 to test a sample(s) in a corresponding target analysis module, manual dispatching is not needed, and accurate and efficient dispatching of the sample during test is further achieved, which further improves test efficiency of the sample and makes use experiences of the product better.

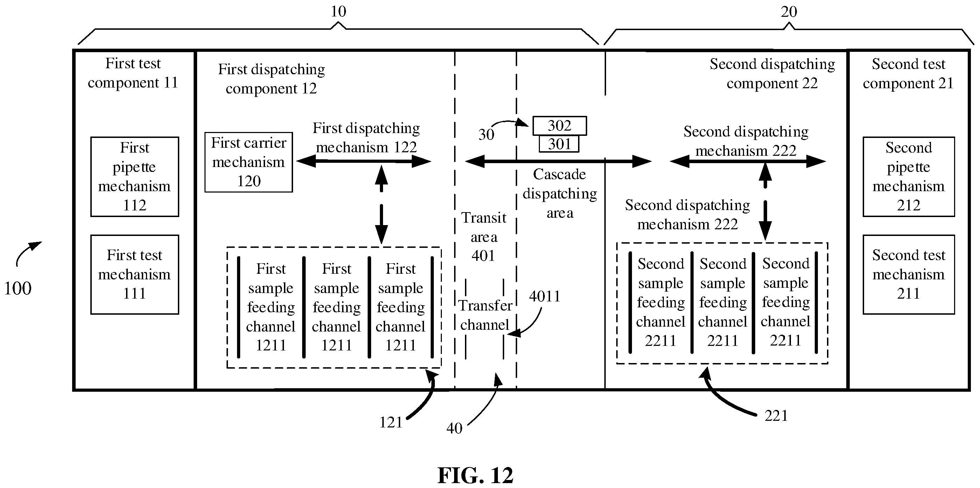

With reference to FIG. 12, in some implementations, the sample analysis system 100 further includes a transit mechanism 40, the transit mechanism 40 is arranged in the first dispatching component 12 and formed with a transit area 401, the first dispatching mechanism 122 is further configured to dispatch at least a portion of sample vessels provided by the first sample feeding mechanism to the transit area 401, and the cascade dispatching mechanism 30 is further configured to dispatch at least a portion of the sample vessels in the transit area 401 to the second dispatching component 22.

Optionally, the first sample feeding mechanism 121 is provided with multiple first sample feeding channels 1211, the transit area 401 is provided with a transfer channel 4011, and at least one of the first sample feeding channels is also used as the transfer channel 4011.

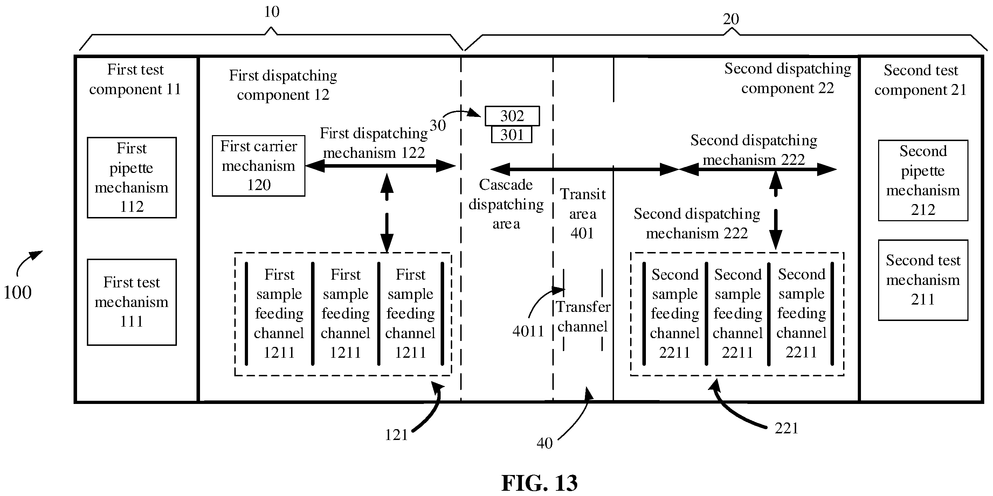

With reference to FIG. 13, in some implementations, the sample analysis system further includes a transit mechanism 401. The transit mechanism 401 is arranged in the second dispatching component 22 and formed with a transit area 401. The second dispatching mechanism 222 is further configured to dispatch at least a portion of the sample vessels provided by the second sample feeding mechanism to the transit area 401. The cascade dispatching mechanism 30 is further configured to dispatch at least a portion of the sample vessels in the transit area 401 to the first dispatching component 12.

Optionally, the second sample feeding mechanism 221 is provided with multiple second sample feeding channels 2211. The transit area 401 is provided with a transfer channel 4011, and at least one of the second sample feeding channels 2211 is also used as the transfer channel 401.

In some implementations, the sample analysis system 100 includes a first test component 11, a first dispatching component 12, a second test component 21 and a second dispatching component 22. At least, the first test component 11 and the first dispatching component 12 form a first analysis module 10 for sample test, and the second test component 21 and the second dispatching component 22 form a second analysis module 20 for sample test.

The first dispatching component 12 and the second dispatching component 22 may dispatch the sample vessels independently of each other, and the first dispatching component 12 and the second dispatching component 22 are put together in a first direction. At least one of the first dispatching component 12 and the second dispatching component 22 is also used as a cascade dispatching mechanism. That is, the first dispatching mechanism 122 is further configured to dispatch at least a portion of sample vessels provided by the second sample feeding mechanism 221 to a first carrier mechanism 120 in the first dispatching component 12, and/or, the second dispatching mechanism 222 is further configured to dispatch at least a portion of sample vessels provided by the first sample feeding mechanism 121 to a second carrier mechanism 220 in the second dispatching component 22. Therefore, sample vessels loaded by two different sample feeding mechanisms may be tested in at least one of the first analysis module 10 and the second analysis module 20 in the sample analysis system, without manual assistance.

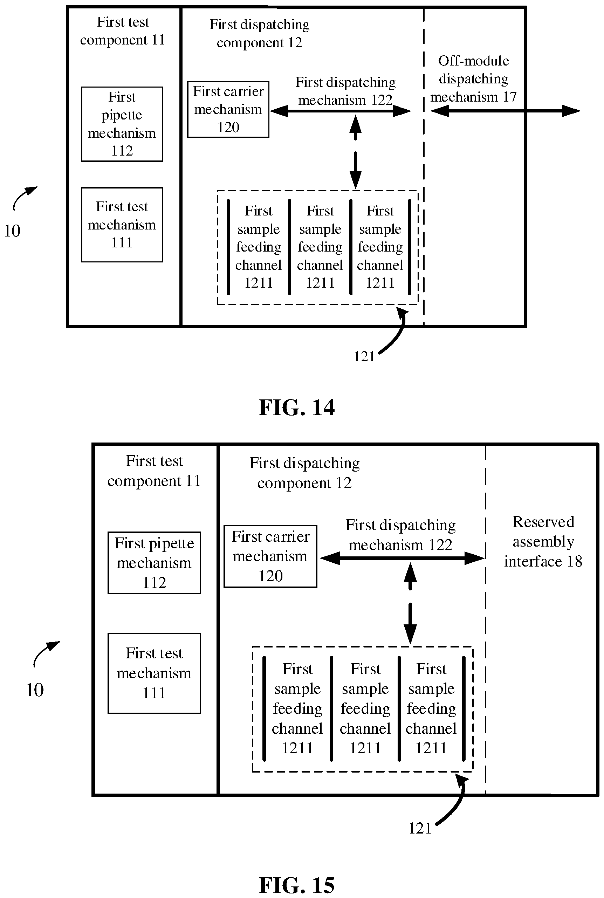

With reference to FIG. 14, in some implementations, the first dispatching component 12 of the first analysis module 10 further includes an off-module dispatching mechanism 17, and a movement trajectory of the off-module dispatching mechanism 17 reaches exterior of the first analysis module 10.

That is, the first analysis module 10 includes the first dispatching component 12 and the first test component 11. The first dispatching component 12 is configured to dispatch at least a portion of sample vessels loaded from the first analysis module 10 to the first test component 11, so as for the first test component 11 to perform test to obtain a first test result of the sample(s). The second analysis module 20 includes the second dispatching component 22 and the second test component 21. The second dispatching component 22 is configured to dispatch at least a portion of sample vessels loaded from the second analysis module 20 to the second test component 21, so as for the second test component 21 to perform test to obtain a second test result of the sample(s).

The first dispatching component 12 includes the first sample feeding mechanism 121, the first dispatching mechanism 122 and the off-module dispatching mechanism 17, the first sample feeding mechanism 121 is provided with a first sample feeding channel 1211, the first sample feeding channel 1211 is configured for the user to place one or more sample vessels that each sample vessel contains a sample, the first test component 11 is configured to test at least a portion of the sample in each of at least a portion of the sample vessels provided by the first sample feeding mechanism 121 to obtain a first test result, and the first dispatching mechanism 122 is configured to dispatch, in the first analysis module 10, at least a portion of sample vessels provided by the first sample feeding mechanism 121, so as for the first test component 11 to test at least a portion of the sample in each of at least a portion of the sample vessels provided by the first sample feeding mechanism 121 to obtain the first test result.

Further, the movement trajectory of the off-module dispatching mechanism 17 may reach exterior of the first analysis module 10, to dispatch the sample vessels loaded from the first sample feeding mechanism 121 to exterior of the first analysis module 10, or dispatch sample vessels loaded from outside the first analysis module 10 to the first analysis module 10 for analysis.

For example, after the first analysis module 10 is connected and cascade-connected with other analysis modules, the off-module dispatching mechanism 17 may be used as the cascade dispatching mechanism to dispatch sample vessels between the first analysis module 10 and other analysis modules, such that the sample loaded from the first analysis module 10 may be tested in other analysis modules, or samples loaded from other analysis modules may be tested in the first analysis module 10.

As may be seen from the above technical solution provided in the present application, the analysis module provided in the present application is provided with two dispatching mechanisms, one of the dispatching mechanisms may dispatch samples in the analysis module, and the other one of the dispatching mechanisms may move to exterior of the analysis module, such that after the analysis module is cascade-connected with another analysis module, mutual dispatching of samples between the analysis module and the other analysis module is achieved. If a user already has an analysis module, and needs a cascade instrument, the user only needs to purchase a corresponding cascade analysis module and put the corresponding cascade analysis module and the analysis module together, it is unnecessary for the user to re-purchase the cascade instrument, which improves use experiences of the user.

With reference to FIG. 15, in some implementations, the first analysis module 10 includes the first dispatching component 12, the first test component 11 and a reserved assembly interface 18. The reserved assembly interface 18 is configured to install an off-module dispatching mechanism, and a movement trajectory of the off-module dispatching mechanism may reach exterior of the first analysis module.

As may be seen from the above technical solution provided in the present application, the analysis module provided in the present application is provided with a reserved assembly interface, such that after the analysis module is cascade-connected with another analysis module, a cascade dispatching mechanism is installed through the reserved assembly interface, and mutual dispatching of samples between the analysis module and the another analysis module is achieved.

If a user already has an analysis module, and needs a cascade instrument, the user only needs to purchase a corresponding cascade analysis module and connect the same with the analysis module, and install a corresponding installation off-module dispatching mechanism (such as the cascade dispatching mechanism) through the reserved assembly interface, it is unnecessary for the user to re-purchase the cascade instrument, which improves use experiences of the product.

It should be understood that terms used in the description of the present application are only for the purpose of describing specific embodiments, and are not intended to limit the present application. As used in the description and appended claims of the present application, singular forms "a", "an" and "the" are intended to include plural forms, unless otherwise indicated clearly in the context.

It should also be understood that a term "and/or" as used in the description and appended claims of the present application refers to any and all possible combinations of one or more of items listed with association, and includes these combinations. It should be noted that terms "include", "including" or any other variants thereof as used here are intended to encompass a non-exclusive inclusion, such that a process, method, article or system including a list of elements includes not only these elements, but also other elements which are not explicitly listed or elements inherent to the process, method, article or system. Without further limitation, an element defined by a statement "including a..." does not preclude presence of additional identical elements in a process, method, article or system including the element.

The above serial numbers of the embodiments of the present application are only for description, and do not represent advantages and disadvantages of the embodiments. The above descriptions are only specific implementations of the present application, however, the scope of protection of the present application is not limited thereto. Various equivalent modifications or replacements may be easily conceived by any technician familiar with this technical field within the technical scope disclosed in the present application, and these modifications or replacements shall fall within the scope of protection of the present application. Therefore, the scope of protection of the present application should be subject to the scope of protection of the claims.

## Claims

1. A sample analysis system (100), **characterized by**, comprising:
a first analysis module (10), comprising a first dispatching component (12) and a first test component (11), wherein the first dispatching component (12) comprises a first sample feeding mechanism (121) and a first dispatching mechanism (122), the first sample feeding mechanism (121) is configured for a user to place one or more first sample vessels that each first sample vessel contains a first sample, the first test component (11) is configured to test at least a portion of the first sample in each of at least a portion of the first sample vessels provided by the first sample feeding mechanism (121), so as to obtain a first test result, and the first dispatching mechanism (122) is configured to dispatch, in the first analysis module (10), at least a portion of the first sample vessels provided by the first sample feeding mechanism (121), so as for the first test component (11) to test at least a portion of the first sample in each of at least a portion of the first sample vessels provided by the first sample feeding mechanism (121), to obtain the first test result;
a second analysis module (20), comprising a second dispatching component (22) and a second test component (21), wherein the second dispatching component (22) comprises a second sample feeding mechanism (221) and a second dispatching mechanism (222), the second sample feeding mechanism (221) is configured for the user to place one or more second sample vessels that each second sample vessel contains a second sample, the second test component (21) is configured to test at least a portion of the second sample in each of at least a portion of the second sample vessels provided by the second sample feeding mechanism (221), so as to obtain a second test result, and the second dispatching mechanism (222) is configured to dispatch, in the second analysis module (20), at least a portion of the second sample vessels provided by the second sample feeding mechanism (221), so as for the second test component (21) to test at least a portion of the second sample in each of at least a portion of the second sample vessels provided by the second sample feeding mechanism (221), to obtain the second test result, the first dispatching component (12) and the second dispatching component (22) dispatch respective sample vessels independently of each other; and
a transit mechanism (40), formed with a transit area (401), wherein the second dispatching mechanism (222) is further configured to dispatch first and/or second sample vessels between the transit area (401) and the second analysis module (20), and the first dispatching mechanism (122) is further configured to dispatch first and/or second sample vessels between the first analysis module (10) and the transit area (401).

2. The sample analysis system (100) of claim 1, wherein the transit mechanism (40) is arranged in the first dispatching component (12), wherein
the first dispatching mechanism (122) is further configured to dispatch at least a portion of the first sample vessels provided by the first sample feeding mechanism (121) to the transit area (401), and the second dispatching mechanism (222) is further configured to dispatch the first sample vessels in the transit area (401) to the second analysis module (20), so as for the second test component (21) to test at least a portion of the first sample in each of the first sample vessels dispatched by the second dispatching mechanism (222), to obtain the second test result, and/or
the second dispatching mechanism (222) is further configured to dispatch at least a portion of the second sample vessels fed by the second sample feeding mechanism (221) to the transit area (401), and the first dispatching mechanism (122) is further configured to dispatch, in the first analysis module (10), the second sample vessels in the transit area (401), so as for the first test component (11) to test at least a portion of the second sample in each of the second sample vessels dispatched by the first dispatching mechanism (122), to obtain the first test result.

3. The sample analysis system (100) of claim 2, wherein the first sample feeding mechanism (121) is formed with a plurality of first sample feeding channels (1211) arranged in a first horizontal direction and extending in a second horizontal direction, so as for the user to insert a first sample holder into one of the first sample feeding channels along the second horizontal direction, and the first sample holder is configured to hold the first sample vessels, wherein
the transit area (401) is formed with at least one transfer channel (4011) extending in the second horizontal direction is provided in the transit area of the transit mechanism to accommodate second sample vessels provided by the second dispatching mechanism (222), and the transfer channel (4011) and the first sample feeding channels (1211) are arranged in a first horizontal direction; and the second horizontal direction is perpendicular to the first horizontal direction.

4. The sample analysis system (100) of claim 2, wherein the first sample feeding mechanism (121) is formed with a plurality of first sample feeding channels (1211) arranged in a first horizontal direction and extending in a second horizontal direction, so as for the user to insert a first sample holder into one of the first sample feeding channels along the second horizontal direction, and the first sample holder is configured to hold the first sample vessels, wherein
the transit area (401) is formed with at least one transfer channel (4011) extending in the second horizontal direction, and, and at least one of the first sample feeding channels (1211) is also used as the transfer channel (4011).

5. The sample analysis system (100) of claim 3 or 4, wherein the first dispatching component (122) comprises a first side and a second side in the second horizontal direction, the first sample feeding channels (1211) comprise a sample vessel inlet (1212) close to the first side and a sample vessel outlet (1213) close to the second side, and a baffle (4012) is arranged at an end of the transfer channel (4011) close to the first side, so as to shield an opening of the transfer channel (4011) close to the first side.

6. The sample analysis system (100) of claim 3 or 4, wherein the transfer channel (4011) is located at a side of the first dispatching component (12) close to the second dispatching component (22).

7. The sample analysis system (100) of claim 3 or 4, wherein the sample analysis system (100) further comprises a control device configured to:
control the first dispatching mechanism (122) to dispatch first and/or second sample vessels in the transfer channel (4011) to a first buffer area (124) in the first dispatching component (12), or control an alarm device (60) of the sample analysis system (100) to output first alarm information, in response to detecting that at least one first and/or second sample vessel is placed by the user in the transfer channel (4011).

8. The sample analysis system (100) of claim 3 or 4, wherein the sample analysis system (100) further comprises a control device configured to:
control the alarm device (60) of the sample analysis system (100) to output second alarm information, in response to detecting that at least one first and/or second sample vessel is removed by the user from the transfer channel (4011).

9. The sample analysis system (100) of claim 8, wherein the control device is configured to:
determine that at least one first and/or second sample vessel is placed by the user in the transfer channel (4011), in response to detecting that the at least one first and/or second sample vessel is placed in the transfer channel (4011) and each of the first dispatching mechanism (122) and the second dispatching mechanism (222) not placing the at least one first and/or second sample vessel in the transfer channel (4011); or
determine that at least one first and/or second sample vessel is removed by the user from the transfer channel (4011), in response to detecting that the at least one first and/or second sample vessel is removed from the transfer channel (4011) and neither the first dispatching mechanism (122) nor the second dispatching mechanism (222) removing the sample vessels from the transfer channel (4011).

10. The sample analysis system (100) of claim 3 or 4, wherein the sample analysis system (100) further comprises a display and a control device, wherein the control device is configured to control the display to display a user interaction interface comprising a first display area and a second display area, and wherein a schematic arrangement diagram of the first sample feeding channels (1211) and the transfer channel (4011) is displayed in the first display area, a schematic arrangement diagram of the first sample feeding channels (1211) and the transfer channel (4011) is displayed in the second display area, the first display area and the second display area do not overlap with each other at least partially, and display parameters of the transfer channel (4011) are different from display parameters of the first sample feeding channels (1211).

11. The sample analysis system (100) of claim 1, wherein the first analysis module (10) further comprises a first scanning mechanism (123), the first test component (11) further comprises a first pipette mechanism (112) and a first test mechanism (111), the first dispatching component (12) is further provided with a first scanning area (14) and a first sample aspiration area (15), wherein
the first scanning mechanism (123) is configured to scan first and/or second sample vessels in the first scanning area (14) to acquire sample information of samples carried by the first and/or second sample vessels, the first pipette mechanism (112) is configured to transfer at least a portion of the sample in each of the first and/or second sample vessels in the first sample aspiration area (15) to the first test mechanism (111), so as for the first test component (11) to test the sample, to obtain the first test result, and
the first dispatching mechanism (122) is further configured to transfer at least a portion of the first sample vessels provided by the first sample feeding mechanism (121) to the first scanning area (14) for scanning, and dispatch at least a portion of the scanned first sample vessels to the first sample aspiration area (15).

12. The sample analysis system (100) of claim 11, wherein the first dispatching component (12) is provided with a first test buffer area (16), and the first dispatching mechanism (122) is further configured to transfer at least a portion of the scanned first sample vessels to the first test buffer area (16) and buffer the scanned first sample vessels in the first test buffer area (16), and dispatch the first sample vessels buffered in the first test buffer area (16) to the first sample aspiration area (15), and
the transit mechanism (40) is arranged in the first test buffer area (16), or the transit mechanism (40) is arranged in the first scanning area (14).

13. The sample analysis system (100) of claim 1, wherein the first analysis module (10) is provided with a first transit part (41), and the second analysis module (20) is provided with a second transit part (42), wherein
the first transit part (41) is arranged in the first dispatching component (12), the second transit part (42) is arranged in the second dispatching component (22), and the first transit part (41) and the second transit part (42) are put together to form a transit mechanism (40) of the sample analysis system (100) with a transit area (401); the second dispatching mechanism (222) is configured to dispatch first and/or second sample vessels between the transit area (401) and the second dispatching component (22), and the first dispatching mechanism (122) is configured to dispatch first and/or second sample vessels between the transit area (401) and the first dispatching component (12), wherein the first dispatching mechanism (122) is further configured to dispatch at least a portion of the first sample vessels provided by the first sample feeding mechanism (121) to the transit area (401), and the second dispatching mechanism (222) is further configured to dispatch the first sample vessels in the transit area (401) to the second analysis module (20), so as for the second test component (21) to test at least a portion of the first sample in each of the first sample vessels dispatched by the second dispatching mechanism (222), to obtain the second test result; or
the second dispatching mechanism (222) is further configured to dispatch at least a portion of the second sample vessels provided by the second sample feeding mechanism (221) to the transit area (401), and the first dispatching mechanism (122) is further configured to dispatch the second sample vessels in the transit area (401) to the first analysis module (10), so as for the first test component (11) to test at least part of the second sample in each of the second sample vessels dispatched by the first dispatching mechanism (122), to obtain the first test result.

14. The sample analysis system (100) of claim 1, wherein the first dispatching component (12) and the second dispatching component (22) are put together in a first horizontal direction, and
wherein the sample analysis system (100) further comprises a third dispatching mechanism (30), wherein third dispatching mechanism (30) comprises a drive member and a transfer member formed with a transit area (401), the drive member is configured to drive the transfer member to move between the first dispatching component (12) and the second dispatching component (22) in the first horizontal direction.

15. The sample analysis system (100) of claim 1, wherein the first analysis module (10) is detachably connected to the second analysis module (20).
